# EUROPÄISCHE PATENTSCHRIFT

(11) **EP 1 595 876 B1**
(45) Veröffentlichungstag und Bekanntmachung des Hinweises auf die Patenterteilung: **12.12.2007**
(21) Anmeldenummer: 05009301.2
(22) Anmeldetag: 28.04.2005
(51) Int. Cl.: C07D 317/46

(54) **Verfahren zur Herstellung von Difluorbenzo-(1,3)-Dioxol-5-Carbonsäurederivaten**
Process for the preparation of derivatives of difluorobenzo-(1,3)-dioxole-5-carboxylic acid
Procédé de préparation de dérivés des acides de difluorobenzo-1,3-dioxolane carboxyliques

(30) Priorität: 14.05.2004 DE 102004024010
(43) Veröffentlichungstag der Anmeldung: 16.11.2005
(73) Patentinhaber: Saltigo GmbH, 40764 Langenfeld (DE)
(72) Erfinder: Pleschke, Axel Dr., 51069 Köln (DE); Marhold, Albrecht Dr., 51373 Leverkusen (DE)
(74) Vertreter: Wichmann, Birgid

(56) Entgegenhaltungen:
- DE-A1- 10 038 019

## Beschreibung

Die Erfindung betrifft ein Verfahren zur Herstellung von Difluorbenzo-[1,3]-dioxol-5-carbonsäure und ihren Derivaten, sowie deren Verwendung zur Herstellung von Arzneimitteln und Pflanzenschutzmitteln.

Difluorbenzodioxol-5-carbonsäure und ihre Derivate spielen eine wichtige Rolle als Vorprodukte für agrochemische und pharmazeutische Wirkstoffe, siehe auch US 4,895,871.

DE 10038019 offenbart ein Verfahren, in dem 2,2-Difluor-benzo[1,3]dioxol mit Urotropin und Hydrogenfluorid zu 2,2-Difluor-benzo[1,3]dioxol-5-carboxaldehyd umgesetzt wird, worauf das erhaltene Aldehyd in das entsprechende Carbonsäurechlorid überführt wird.

Bislang sind zwei Verfahren zur Herstellung von Difluorbenzo-[1,3]-dioxol-5-carbonsäure und ihren Derivaten bekannt. So beschreibt US 4,895,871 eine zweistufige Synthesesequenz ausgehend von Difluorbenzo-[1,3]-dioxol, die einen Bromierungsschritt sowie eine Lithiierung, Quenchung mit Kohlendioxid und wässrig-saure Aufarbeitung vorsieht. Aufgrund der hochsensitiven lithiumorganischen Zwischenverbindungen ist dieses Verfahren für eine industrielle Anwendung unpraktikabel. Weiterhin ist aus Yagupolski, Troitskaya, Russ. J. Org. Chem., 30.9.1960, 3129-3132 bekannt, Benzodioxol-5-carbonsäure zunächst mit Phosphorpentachlorid zu Dichlorbenzodioxol-5-carbonsäurechlorid zu chlorieren und die weitere Umsetzung zu Difluorbenzo-[1,3]-dioxol-5-carbonsäurefluorid mit Antimontrifluorid durchzuführen. Aufgrund des hohen Preises und der Toxizität von Antimontrifluorid ist auch dieses Verfahren im industriellen Maßstab nicht anwendbar.

Es bestand also der Bedarf nach einem Verfahren, das die Herstellung von Difluorbenzo-[1,3]-dioxol-5-carbonsäure und ihren Derivaten ausgehend von einfach verfügbaren Edukten auch in technischem Maßstab ermöglicht.

Es wurde nun ein Verfahren zur Herstellung von Verbindungen der Formel (I) gefunden, 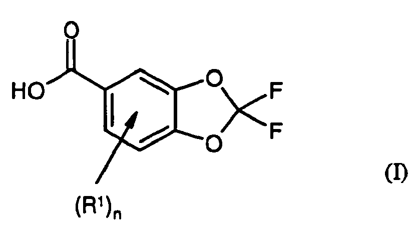 in der
- R¹: für C₁-C₁₂-Alkyl, C₁-C₁₂-Fluoralkyl, C₁-C₁₂-Alkoxy, C₁-C₁₂-Fluoralkoxy, Brom, Chlor oder Fluor und
- n: für 0, 1, 2 oder 3 steht,
das dadurch gekennzeichnet ist, dass Verbindungen der Formel (II) 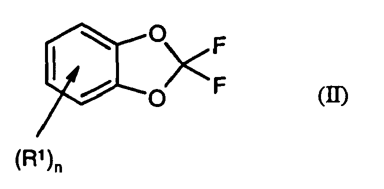 in Gegenwart
- von Bortrifluorid und
- Fluorwasserstoff
zunächst zu Verbindungen der Formel (III) umgesetzt werden, 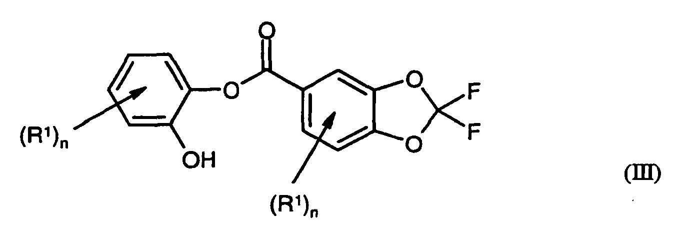 und die Verbindungen der Formel (III) gegebenenfalls nach zumindest teilweisem Abdestillieren von Bortrifluorid und/oder Fluorwasserstoff
in wässrig-saurem oder alkalischem Milieu zu Verbindungen der Formel (I) hydrolysiert werden.

Der Rahmen der Erfindung umfasst alle oben stehenden und im Folgenden aufgeführten, allgemeinen oder in Vorzugsbereichen genannten Restedefinitionen, Parameter und Erläuterungen untereinander, also auch zwischen den jeweiligen Bereichen und Vorzugsbereichen in beliebiger Kombination.

**Alkyl** beziehungsweise **Alkoxy** steht jeweils unabhängig für einen geradkettigen, cyclischen, verzweigten oder unverzweigten Alkyl- beziehungsweise Alkoxy-Rest, wobei die genannten Reste gegebenenfalls durch C₁-C₄-Alkoxy-Reste weiter substituiert sein können.

C₁-C₁₂-Alkyl steht beispielsweise und bevorzugt für Methyl, Ethyl, n-Propyl, iso-Propyl, n-Butyl, sec.-Butyl, tert.-Butyl, n-Pentyl, 1-Methylbutyl, 2-Methylbutyl, 3-Methylbutyl, neo-Pentyl, 1-Ethylpropyl, cyclo-Hexyl, cyclo-Pentyl, n-Hexyl, n-Heptyl, n-Octyl, n-Decyl und n-Dodecyl.

C₁-C₁₂-Alkoxy steht beispielsweise und bevorzugt für Methoxy, Ethoxy, n-Propoxy, iso-Propoxy, n-Butoxy, sec.-Butoxy, tert.-Butoxy, n-Pentoxy, cyclo-Hexoxy, cyclo-Pentoxy, n-Hexoxy, n-Heptoxy, n-Octoxy, n-Decoxy und n-Dodecoxy

**Fluoralkyl** beziehungsweise **Fluoralkoxy** steht jeweils unabhängig für einen geradkettigen, cyclischen, verzweigten oder unverzweigten Alkyl-Rest beziehungsweise Alkoxy-Rest, der einfach, mehrfach oder vollständig durch Fluoratome substituiert ist.

Beispielsweise steht C₁-C₁₂-Fluoralkyl für Trifluormethyl, 2,2,2-Trifluorethyl, Pentafluorethyl, Nonafluorbutyl, Heptafluorisopropyl, Perfluoroctyl und Perfluordodecyl.

Beispielsweise steht C₁-C₁₂-Fluoralkoxy für Trifluormethoxy, 2,2,2-Trifluorethoxy, Pentafluorethoxy, Nonafluorbutoxy, Heptafluorisopropoxy, Perfluoroctoxy und Perfluordodecoxy.

Im Folgenden werden die bevorzugten Substitutionsmuster definiert:
- R¹: steht besonders bevorzugt für Methyl, Ethyl, n-Propyl, Chlor, Fluor und Brom, ganz besonders bevorzugt für Chlor oder Fluor.
- n: steht bevorzugt für 0, 1 oder 2, besonders bevorzugt für 0 oder 1 und ganz besonders bevorzugt für 0.

Bevorzugte Verbindungen der Formel (II) sind 2,2-Difluorbenzo-[1,3]-dioxol 2,2-Difluor-5-chlorbenzo)-[1,3]-dioxol und 2,2-difluor-(5-brombenzo)-[1,3]-dioxol.

Bevorzugte Verbindungen der Formel (I) sind 2,2-Difluorbenzo-[1,3]-dioxol-5-carbonsäure,6-Chlor-2,2-Difluor-benzo-[1,3]-dioxol-5-carbonsäure und 2,2-Difluor-(6-brombenzo)[1,3]-dioxol-5-carbonsäure.

Die Verbindungen der Formel (III) sind bislang nicht beschrieben und daher von der Erfindung als unverzichtbare Intermediate ebenfalls umfasst. Die Bedeutung einschließlich der genannten Vorzugsbereiche gilt dabei für R¹ und n in gleicher Weise.

Bevorzugte Verbindungen der Formel (III) sind 2,2-Difluorbenzo-[1,3]-dioxol-5-carbonsäure-2-hydroxyphenylester, 6-Chlor-2,2-difluor-benzo[1,3]dioxol-5-carbonsäure-5-chlor-2-hydroxy-phenylester und 5-Brom-2,2-Difluor-benzo[1,3]-dioxol-5-carbonsäure-2-hydroxyphenylester.

Das erfindungsgemäße Verfahren wird in Gegenwart von Bortrifluorid und Fluorwasserstoff durchgeführt. Dabei ist für den Fachmann klar, dass sich die beiden Komponenten zu Tetrafluoroborsäure verbinden, ohne dass dies jeweils gesondert erwähnt wird.

Das molare Verhältnis von Bortrifluorid zu Verbindungen der Formel (I) kann beispielsweise 0,1 bis 10 betragen, bevorzugt jedoch 0,3 bis 3 und besonders bevorzugt 0,4 bis 0,7. Größere Mengen sind möglich, jedoch unwirtschaftlich.

Das molare Verhältnis von Fluorwasserstoff zu Verbindungen der Formel (I) kann beispielsweise 0,1 bis 100 betragen, bevorzugt jedoch 1 bis 30 und besonders bevorzugt 2 bis 15. Größere Mengen sind möglich, jedoch unwirtschaftlich.

Die Reaktionstemperatur kann im ersten Schritt beispielsweise -30 bis 100°C betragen, bevorzugt 0 bis 50°C, der Reaktionsdruck 0,5 bis 100 bar, bevorzugt 0,9 bis 12 bar.

Die Dosierabfolge zwischen Verbindung der Formel (I), Bortrifluorid und Fluorwasserstoff ist beliebig, aus Praktikabilitätsgründen wird jedoch vorzugsweise Fluorwasserstoff mit Bortrifluorid und anschließend mit Verbindung der Formel (I) versetzt oder Verbindung der Formel (I) zunächst mit Fluorwasserstoff und dann mit Bortrifluorid.

Die Verbindungen der Formel (III) die im ersten Schritt entstehen werden vorzugsweise nach zumindest teilweisem Abdestillieren von Bortrifluorid und/oder Fluorwasserstoff in wässrig saurem oder alkalischem Milieu zu Verbindungen der Formel (I) hydrolysiert.

Teilweise bedeutet dabei bevorzugt das Abdestillieren von 60 bis 98 % des überschüssigen Bortrifluorid und/oder Fluorwasserstoffs.

Vorzugsweise erfolgt die Hydrolyse in wässrig alkalischem Milieu, wobei wie allgemein üblich wässrig alkalisch einen pH-Wert von über 7 bei 25°C bedeutet. Zur Hydrolyse können dabei beispielsweise Alkalimetall- oder Erdalkalimetallhydroxiden oder -carbonaten, bevorzugt Alkalimetallhydroxyden oder jeweils wässrige Lösungen davon verwendet werden, besonders bevorzugt ist eine 1 bis 50 gew.-% ige wässrige Lösung von Kaliumhydroxid.

Bei der alkalischen Hydrolyse entstehen zunächst die der Formel (III) entsprechenden Phenolate, die ebenfalls von der Erfindung umfasst sind, bevor sich die Carbonsäuresalze von Verbindungen der Formel (I) bilden.

Zur Freisetzung der freien Carbonsäuren gemäß Formel (I) wird bei alkalischer Hydrolyse vorzugsweise angesäuert, und zwar mit einer Säure die bei 25°C einen pKa-Wert besitzt, der höher, vorzugsweise mindestens 2 Einheiten höher ist, als der der freizusetzenden Säure. Beispielsweise kann gegebenenfalls mit Wasser verdünnte Schwefelsäure verwendet werden.

Auf erfindungsgemäße Weise können Verbindungen der Formel (I) in hoher Reinheit und Ausbeute in einfacher Weise erhalten werden.

Die Erfindung umfasst weiterhin ein Verfahren zur Herstellung von Verbindungen der Formel (IV) 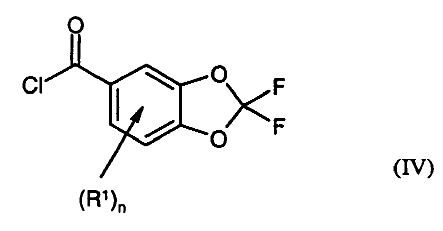 in der
- R¹ und n: die oben genannten Bedeutungen und Vorzugsbereiche besitzen,
das dadurch gekennzeichnet ist dass es das vorstehend beschriebene Verfahren und als weiteren Schritt die Umsetzung mit einen Chlorierungsmittel umfasst.

Als Chlorierungsmittel eignen sich beispielsweise und insbesondere Thionylchlorid, Phosgen und Phosphorylchlorid.

Die erfindungsgemäßen Verbindungen der Formel (III) und deren Phenolate eignen sich insbesondere zur Anwendung in einem Verfahren zur Herstellung von Arzneimitteln, Agrochemikalien oder Zwischenprodukten davon.

### Beispiele:

### Beispiel 1:

### 2,2-Difluorbenzo[1,3]dioxol-5-carbonsäure 2-hydroxyphenyl ester:

880 ml HF werden bei 0°C vorgelegt und 259 g BF3 innerhalb 30 min aufgepresst. Anschließend werden 1000 g Difluorbenzodioxol innerhalb 30 min. zugegeben und 1 bar Stickstoff aufgedrückt. Die Temperatur wird innerhalb 30 min auf 15°C gesteigert und anschließend wird 10 Stunden bei 15°C nachgerührt.

Der Ansatz wird auf 1500 g Eiswasser gedrückt und die kristalline Ausfällung durch Zugabe von Methylenchlorid gelöst. Die organische Phase wird abgetrennt und mit gesättigter Natriumhydrogencarbonat-Lösung auf pH-5 eingestellt. Wiederum wird die organische Phase abgetrennt, mit Wasser gewaschen, getrocknet und eingeengt. Man erhält 811 g farblosen Feststoff (≙ 43,3 % bzw. 86,6 % bezogen auf eingesetztes Difluorbenzodioxol).

¹H-NMR (400 MHz, DMSO-D₆): 9.83 (b, 1 H), 8.07 (m,2 H), 7,59 (m, 1H), 7,15 (m, 2 H), 7.02 (m, 1 H), 6.87 (m, 1H)

### Beispiel 2:

### 2,2-Difluorbenzo-[1,3]-dioxol-5-carbonsäure:

1012 g HF werden bei 0°C vorgelegt und 257 g BF3 innerhalb 60 min aufgedrückt. Anschließend werden 1000 g Difluorbenzodioxol innerhalb 60 Minuten zugegeben und die Temperatur innerhalb 30 min auf 15°C gesteigert. Anschließend wird 16 Stunden bei 15°C nachgerührt. Anschließend werden bei Normaldruck bis 55°C Innentemperatur 773g HF abdestilliert.

5 L KOH-Lösung 10%ig werden vorgelegt und unter Kühlung der Rückstand der Destillation zutropft. Nach beendeter Zugabe wird 6 Stunden bei 50°C nachgerührt, wobei der pH-Wert durch Zugabe von KOH zwischen 10 und 11 gehalten wird. Anschließend wird der Ansatz abkühlt, der Niederschlag abgesaugt und die Mutterlauge mit Schwefelsäure auf pH 5 gestellt. Nach Absaugen und Trocknen erhält man 529 g beigen Feststoff. Ausbeute: 40,7 % ≙ 81,4 % bezogen auf Difluorbenzodioxol

¹H-NMR (400 MHz, DMSO-D₆): 13.52 (b, 1 H), 7.87 (m, 2 H), 7,53 (d, 1H)

### Beispiel 3:

### 6-Chlor-2,2-dilluor-benzo[1,3]dioxol-5-carbonsäure-2-hydroxy-phenyl ester:

830 g HF werden bei 0°C vorlegt und 211 g BF3 innerhalb 30 min aufgepresst. Anschließend werden 1000 g 5-Chlor-difluorbenzodioxol innerhalb 30 min. zugegeben und die Temperatur innerhalb 30 min auf 15°C gesteigert. Anschließend wird 10 Stunden bei 15°C nachgerührt.

Der Ansatz wird auf 1500 g Eiswasser gegeben und die kristalline Ausfällung durch Zugabe von Methylenchlorid gelöst. Die organische Phase wird abgetrennt und mit gesättigter Natriumhydrogencarbonat-Lösung auf pH-5 eingestellt. Wiederum wird die organische Phase abgetrennt, mit Wasser gewaschen, getrocknet und eingeengt. Man erhält 1122 g farblosen Feststoff.

¹H-NMR (400 MHz, DMSO-D₆): 10.32 (b, 1 H), 8.16 (m, 1 H), 7.9 (s, 1 H), 7.22 (d, 1 H), 7.02 (m, 1 H), 6.92 (m,1 H)

### Beispiel 4:

### 6-Chlor-2,2-difluor-benzo[1,3]dioxol-5-carbonsäure:

723 ml HF werden bei 0°C vorgelegt und 212 g BF3 innerhalb 60 min aufgedrückt. Anschließend werden 1000 g 5-Chlordifluorbenzodioxol innerhalb 60 Minuten zugegeben und die Temperatur innerhalb 30 min auf 15°C gesteigert. Anschließend wird 16 Stunden bei 15°C nachgerührt. Danach wird bei Normaldruck bis 55°C Innentemperatur HF abdestilliert.

5L KOH-Lösung 10%ig werden vorgelegt und unter Kühlung der Rückstand der Destillation zugetropft. Nach beendeter Zugabe wird 6 Stunden bei 50°C nachgerührt, wobei der pH durch Zugabe von KOH zwischen 10 und 11 gehalten wird. Der Ansatz wird abgekühlt, der Niederschlag abgesaugt und die Mutterlauge mit Schwefelsäure auf pH 5 gestellt. Nach Absaugen und Trocknen erhält man 466 g schwach beigen Feststoff.

¹H-NMR (400 MHz, DMSO-D₆): 13.52 (b, 1H), 7.68 (s, 1 H), 7,65 (s, 1H)

## Patentansprüche

1. Verfahren zur Herstellung von Verbindungen der Formel (I), 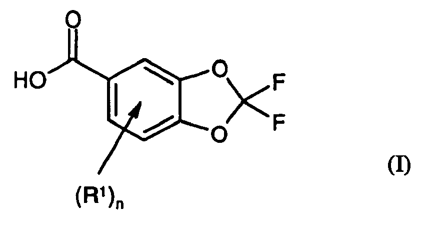 in der
R¹ für C₁-C₁₂-Alkyl, C₁-C₁₂-Fluoralkyl, C₁-C₁₂-Alkoxy, C₁-C₁₂-Fluoralkoxy, Brom, Chlor oder Fluor und
n für 0, 1, 2 oder 3 steht, wobei die genannten Alkyl und Alkoxy-Reste gegebenenfalls durch C₁-C₄ Alkoxy-Reste weiter substituiert sein können,
**dadurch gekennzeichnet, dass** Verbindungen der Formel (II) 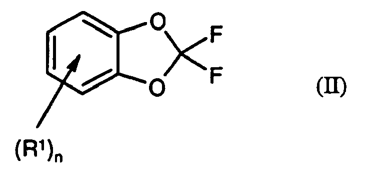 in Gegenwart
• von Bortrifluorid und
• Fluorwasserstoff
zunächst zu Verbindungen der Formel (III) umgesetzt werden, 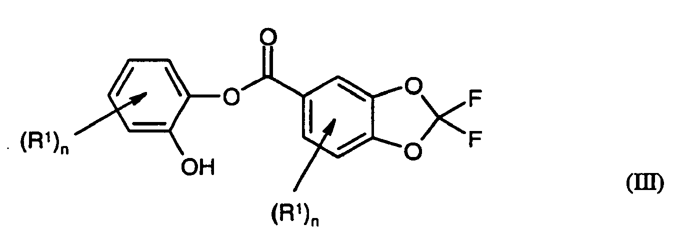 und die Verbindungen der Formel (III) gegebenenfalls nach zumindest teilweisem Abdestillieren von Bortrifluorid und/oder Fluorwasserstoff
in wässrig saurem oder alkalischem Milieu zu Verbindungen der Formel (I) hydrolysiert werden.

2. Verfahren nach Anspruch 1, **dadurch gekennzeichnet, dass** R¹ für Methyl, Ethyl, n-Propyl, Chlor, Fluor und Brom steht und n für 0, 1 oder 2.

3. Verfahren nach Anspruch 1 oder 2, **dadurch gekennzeichnet, dass** als Verbindungen der Formel (II) 2,2-Difluorbenzo-[1,3]-dioxol, 2,2-Difluor-(5-chlorbenzo)-[1,3]-dioxol oder 2,2-Difluor-(brombenzo)-[1,3]-dioxol eingesetzt werden.

4. Verbindungen der Formel (III) und deren Phenolate 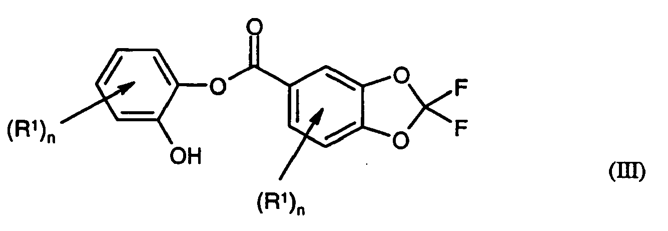 mit der in Anspruch 1 angegebenen Bedeutung.

5. Folgende Verbindungen nach Anspruch 4: 6-Chlor-2,2-difluor-benzo[1,3]dioxol-5-carbonsäure-2-hydroxy-phenylester, 6-Brom-2,2-difluor-benzo[1,3]dioxol-5-carbonsäure-2-hydroxy-phenylester.

6. Verfahren zur Herstellung von Verbindungen der Formel (IV) 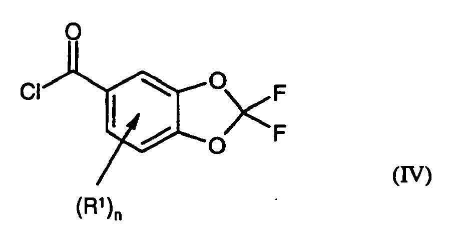 in der
R¹ und n die in Anspruch 1 angegebene Bedeutung besitzen,
**dadurch gekennzeichnet, dass** es
• ein Verfahren nach Anspruch 1 und
• die Umsetzung mit einen Chlorierungsmittel umfasst.

7. Verwendung von Verbindungen nach Anspruch 4 oder 5 in einem Verfahren zur Herstellung von Arzneimitteln, Agrochemikalien oder Zwischenprodukten davon.

## Claims

1. Process for preparing compounds of the formula (I) 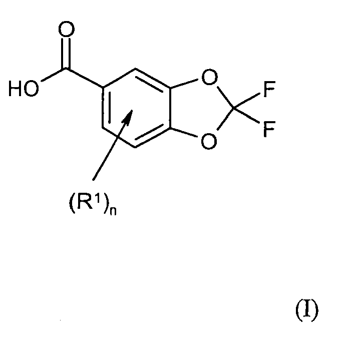 in which
R¹ is C₁-C₁₂-alkyl, C₁-C₁₂-fluoroalkyl, C₁-C₁₂-alkoxy, C₁-C₁₂ -fluoroalkoxy, bromine, chlorine or fluorine and
n is 0, 1, 2 or 3, where the alkyl and alkoxy radicals mentioned may optionally be further substituted by C₁-C₄-alkoxy radicals,
**characterized in that** compounds of the formula (II) 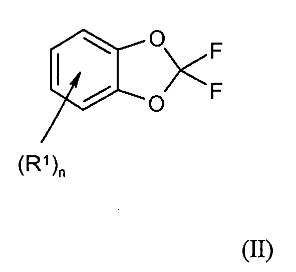 are reacted in the presence
• of boron trifluoride and
• hydrogen fluoride
initially to give compounds of the formula (III) 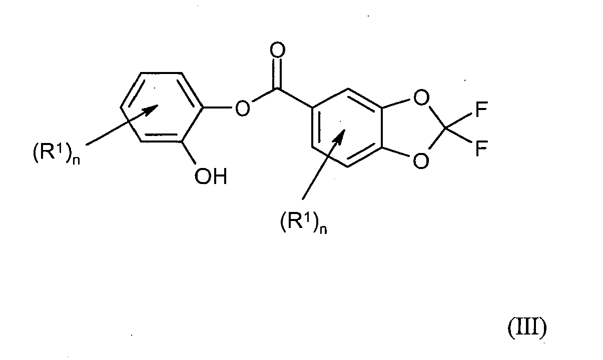 and the compounds of the formula (III), optionally after at least partial removal of boron trifluoride and/or hydrogen fluoride by distillation,
are hydrolysed in aqueous acidic or alkaline medium to give compounds of the formula (I).

2. Process according to Claim 1, **characterized in that** R¹ is methyl, ethyl, n-propyl, chlorine, fluorine and bromine, and n is 0, 1 or 2.

3. Process according to Claim 1 or 2, **characterized in that** the compounds of the formula (II) used are 2,2-difluorobenzo-1,3-dioxole, 2,2-difluoro-5-chlorobenzo-1,3-dioxole or 2,2-difluoro-5-bromobenzo-1,3-dioxole.

4. Compounds of the formula (III) and phenoxides thereof 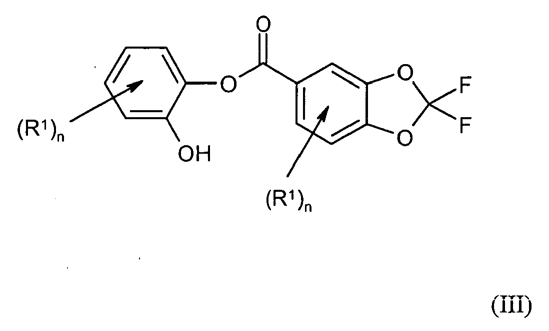 with the definitions specified in Claim 1.

5. The following compounds according to Claim 4: 2-hydroxyphenyl 6-chloro-2,2-difluorobenzo-1,3-dioxole-5-carboxylate, 2-hydroxyphenyl 6-bromo-2,2-difluorobenzo-1,3-dioxole-5-carboxylate.

6. Process for preparing compounds of the formula (IV) 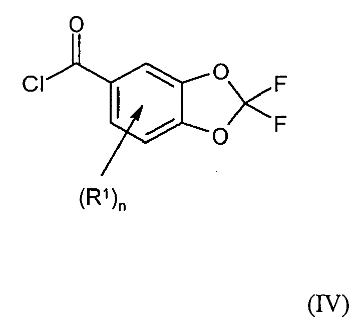 in which
R¹ and n are each as defined in Claim 1,
**characterized in that** it comprises
• a process according to Claim 1 and
• the reaction with a chlorinating agent.

7. Use of compounds according to Claim 4 or 5 in a process for preparing medicaments, agrochemicals or intermediates thereof.

## Revendications

1. Procédé de préparation de composés de formule (I), 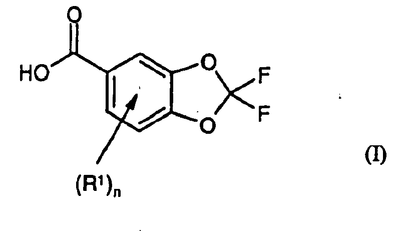 dans laquelle
R¹ représente un alkyle en C₁-C₁₂, un fluoroalkyle en C₁-C₁₂, un alcoxy en C₁-C₁₂, un fluoroalcoxy en C₁-C₁₂, le brome, le chlore ou le fluor, et
n représente 0, 1, 2 ou 3, les radicaux mentionnés alkyle et alcoxy pouvant encore être le cas échéant substitués par des radicaux alcoxy en C₁-C₄,
**caractérisé en ce que** les composés de formule (II) 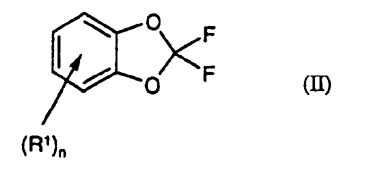 sont mis à réagir en présence
• de trifluorure de bore et
• de fluorure d'hydrogène
d'abord pour obtenir des composés de formule (III) 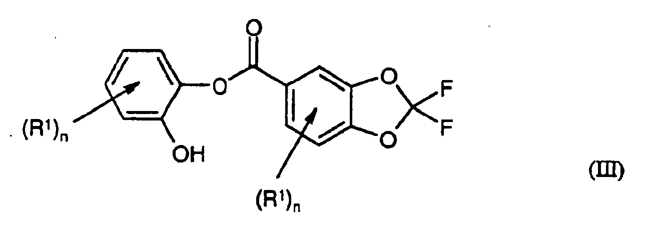 et les composés de formule (III) sont le cas échéant, après séparation par distillation au moins partielle du trifluorure de bore et/ou du fluorure d'hydrogène,
sont hydrolysés en milieu aqueux acide ou alcalin pour obtenir les composés de formule (I).

2. Procédé selon la revendication 1, **caractérisé en ce que** R¹ représente méthyle, éthyle, n-propyle, le chlore, le fluor et le brome et n est 0, 1 ou 2.

3. Procédé selon la revendication 1 ou 2, **caractérisé en ce qu'**on utilise comme composés de formule (II), du 2,2-difluorobenzo-[1,3]dioxole, du 2,2-difluoro-(5-chlorobenzo)-[1,3]-dioxole ou du 2,2-difluoro-(bromobenzo)-[1,3]-dioxole.

4. Composés de formule (III) et leurs phénolates 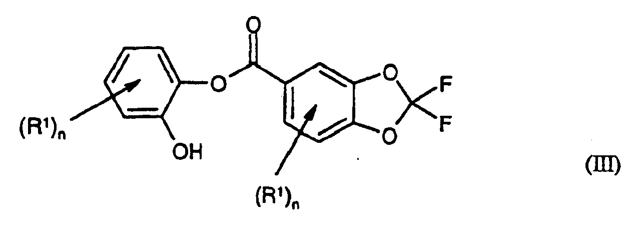 avec la signification donnée à la revendication 1.

5. Composés suivants selon la revendication 4: 2-hydroxy-phénylester d'acide 6-chloro-2,2-difluoro-benzo[1,3]dioxol-5-carboxyliq ue, 2-hydroxy-phénylester d'acide 6-bromo-2,2-difluoro-benzo[1,3]dioxol-5-carboxyliqu e.

6. Procédé de préparation de composés de formule (IV) 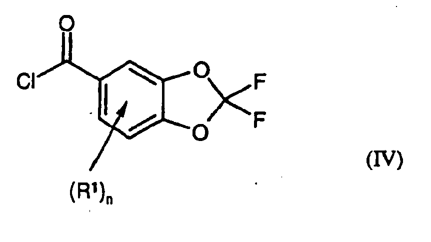 dans laquelle
R¹ et n possèdent la signification donnée à la revendication 1,
**caractérisé en ce qu'**il comprend
• un procédé selon la revendication 1, et
• la réaction avec un agent de chloration.

7. Utilisation de composés selon la revendication 4 ou 5 dans un procédé de préparation de médicaments, de produits agrochimiques ou de produits intermédiaires de ceux-ci.
